# EUROPEAN PATENT APPLICATION

(11) **EP 2 361 614 A2**
(43) Date of publication of application: **31.08.2011**
(21) Application number: 09827689.2
(22) Date of filing: 09.11.2009
(51) Int. Cl.: A61K 9/22, A61K 9/20, A61K 47/02, A61K 47/26, A61K 47/38

(54) **FAST DISSOLVING ORAL TABLETS AND METHOD FOR PRODUCTION THEREOF**

(30) Priority: 21.11.2008 KR 20080116414
(71) Applicant: CJ CheilJedang Corporation, Jung-gu Seoul 100-749 (KR)
(72) Inventor: LEE, Si Beum, Yongin-si Gyeonggi-Do 446-753 (KR); CHO, Il Hwan, Seoul 157-200 (KR); LEE, Go Eun, Seoul 158-753 (KR); AN, Tae Kun, Yongin-si Gyeonggi-Do 446-923 (KR); HONG, Il Ki, Incheon 403-100 (KR); JEONG, Seon Oh, Yongin-si Gyeonggi-Do 449-070 (KR); LEE, Yong Taek, Seoul 153-035 (KR); SHIN, Kyung Min, Seoul 156-093 (KR); YOON, Myeong Sik, Yongin-si Gyeonggi-Do 448-751 (KR)
(74) Representative: Owen, Deborah Jane
(86) International application number: PCT/KR2009/006536
(87) International publication number: WO 2010/058924

(57) **Abstract**

Provided is a fast dissolving oral tablet, and a method for the production thereof, including the steps of: compressing a mixture of active ingredients, pharmaceutically acceptable additives, and supercritical fluid-soluble substances to produce the tablets; and allowing the tablets to contact a supercritical fluid to extract supercritical fluid-soluble substances from fine pores in the tablets.

## Description

### BACKGROUND OF THE INVENTION

### Field of the Invention

The present invention relates to fast dissolving oral tablets and a method for the production thereof, and more particularly, to porous fast dissolving oral tablets that are produced by a supercritical fluid extraction process, and a method for the production thereof.

### Description of Related Art

Generally, a tablet or a capsule should be taken with 200∼250 mL water, but these taking ways may be uncomfortable or impracticable for some patients. Especially, in the case of the aged, babies, children, people who are hard to swallow, patients who should limit their water intake, and patients who are hard to manage due to physical and mental reasons such as dementia, Parkinson's disease, and the like, a tablet is hard to be given to them by using a general way. In this case, preferably, fast dissolving tablets that are easily dissolved by saliva in the mouth or converted in a liquid phase (hereinafter, called as 'fast dissolving tablets') should be given to them. In addition, the fast dissolving tablets are quickly dissolved so that it can be very useful to quickly absorb a physiological active substance, or to increase bioavailability by absorbing the physiological active substance through the mouth and the esophagus.

The fast dissolving tablets are a formulation that are quickly dissolved by saliva in the mouth to release the physiological active substance, or particles containing the physiological active substance, and generally are dissolved within 5 minutes. After developing a technique that is called as Zydis as a trade name produced by freeze-drying from R.P. Scherer company for the first time, the technique that is called as Fast-dissolving began to receive attention and the development of the technique has become faster as launching the product applied with Zydis technique. The fast dissolving tablets should functionally have the features as follows: i) the tablets should be quickly dissolved in the mouth; ii) a water insoluble residue should not be left in the mouth; and iii) it should have a proper mechanical strength in order to reduce damage during handling and distributing.

A method for producing the fast dissolving tablets developed up to the present can be classified into a molding process, a freeze-drying method, a sublimation process, disintegrating agent-adding method, sugar-re-crystallization method, and the like, and only one method or the combination of various methods from the above methods can be used.

US 5,631,023 and US 5,976,577 disclose the fast dissolving tablets formulated by Lyophilization of the solution containing a drug, and the formulation includes Pepcid RPD (Famotidine, Merck), Zofran Zydis (Ondansetron, Glaxo Wellcome), Claritin RediTabs (Loratadine), and the like. The products as mentioned above has an advantage such that they can quickly be dissolved within 2 to 3 seconds in the mouth, but also has disadvantages such that they have low productivity when producing because they should be lyophilized at a specific temperature condition after injecting the drug solution to the pre-molded container, and they have low economical efficiency due to the cost of the production is high because the formulation produced as mentioned above should be packaged with a special material. In addition, there is a disadvantage such that their hardness and friability cannot be measured because the development is too focused on the basic function that allows being able to dissolve quickly in the mouth.

Instead of the lyophilization having the disadvantages as mentioned above, Yamanouchi in Japan developed WOWTAB Technique, and WO 99147126 as a patent document related to the above technique discloses a method for producing the organic solvent-free fast dissolving tablets after tableting an aqueous polymer as a binder with an active component, wetting it under a high humid condition and then drying. In addition, WO 93/12769 discloses a method comprising: filling a suspension such as an active component, agar, sugar, and the like to a mold; and drying it at 30 °C and 760 mmHg to remove water. However, there are disadvantages such that the methods also have low productivity and the products having a steady quality between products are difficult to be obtained.

In addition, US 6,024,981 and 6,221,392 (Orasolv, Durasolv technique, Cima Labs) disclose a method for producing fast dissolving oral tablets by mixing an excipient that can be directly tableted with an active component. The method has an advantage such that the mechanical strength is compensated but can be applied only for the tablets having a small amount of therapeutical component because the excessive amount of a sugar that can be directly tableted or a strong dissolving agent is used. In addition, the method has disadvantage such that it cannot be applied when using a high capacity drug or the tablets grow in size abnormally if it can be applied.

As another method for producing the fast dissolving oral tablets, Cima Labs developed Orasolv technique (US 5,178,878 and US 6,024,981). A representative product formulated by using the Orasolve technique is Zimig Rapimelt (zolmitriptan, Astrazeneca). The product as mentioned above is the tablet containing an epispastics material, but the tablets do not have an oral dissolvability and also a patient's compliance for taking drug is not high due to the generation of epispastics gas in the mouth.

Meanwhile, a supercritical fluid is an incompressible fluid under the temperature and pressure of above critical point, and has excellent physical properties that are not the properties of the conventional organic solvent, such as a density like liquid, a viscosity like gas, a high diffusion coefficient, very low surface tension, and the like, at the same time. Moreover, the supercritical fluid can allow to continuously change the density from a dilute phase that is similar with an ideal gas to high-density state that is similar with liquid density so that it can control an equilibrium property (solubility), a transport property (viscosity, diffusion coefficient, thermal Conductivity), a state of molecules clustering, and the like. Currently, there have actively been plenty of studies in the fields, such as a selective extraction of a special material, an analysis of material through an extracted material, and the like by using the unique properties of supercritical fluid as mentioned above.

### SUMMARY OF THE INVENTION

Therefore, the inventors studied for developing fast dissolving oral tablets in which the fast dissolving oral tablets can be economically produced and also easily dissolved or disintegrated in the mouth while the fast dissolving oral tablets have a sufficient mechanical strength. As a result, the inventors finished the present invention by developing porous fast dissolving tablets having the advantages as mentioned above by using a supercritical fluid extraction process that is the technique for separating a material by using a supercritical fluid as a solvent.

Therefore, an object of the present invention is to provide the fast dissolving oral tablets having excellent mechanical strength, fast dissolving property, and economic feasibility by using the supercritical fluid extraction process.

In addition, another object of the present invention is to provide a method for producing the fast dissolving oral tablets by using the supercritical fluid extraction process, in which the fast dissolving oral tablets have the excellent mechanical strength, fast dissolving properly, and economic feasibility.

The advantages, features and aspects of the invention will become apparent from the following description of the embodiments with reference to the accompanying drawings, which is set forth hereinafter.

In order to achieve the objects, an aspect of the present invention provides fast dissolving oral tablets produced by the method comprising:

producing the tablets by tableting the mixture containing active ingredients, pharmaceutically acceptable additives, and supercritical fluid-soluble substances; and

forming fine pores in the tablets by extracting the supercritical fluid-soluble substances by contacting the tablets with the supercritical fluid.

The fast dissolving oral tablets provided by the present invention is produced by extracting the supercritical fluid-soluble substances by contacting with the supercritical fluid after tableting through mixing the active ingredients, the pharmaceutically acceptable additives, and the supercritical fluid-soluble substances. In addition, the fast dissolving oral tablets have porous pores by removing through the extraction of the supercritical fluid-soluble substances. The fast dissolving oral tablets produced by the process for forming the porous pores will have the fast dissolving property in the mouth by having the porous pores.

Korean Patent Publication No. 2003-0076051 discloses a method for generating pores by mixing a sublimation material and a method for generating pores by contacting with solvent as a method for generating the porous pores during purifying. However, the method for mixing with the sublimation material needs to remove the sublimation material by supplying heat for scores of hours so that it is an energy-consuming process, and also it can effect on the stability of drug so that the temperature and velocity for heating are limited. In addition, the method for forming the pores by contacting with solvent had disadvantages such that the size of tablet grows larger due to the swelling of additives and most of solvents are an organic solvent that is harmful to the environment.

In comparison, for the forming of the porous pores by the supercritical fluid extraction process according to the present invention, the diffusion velocity of the supercritical fluid is fast and the surface tension of the supercritical fluid is low so that it can perform effectively on quickly penetrating to the fine pores between powders and quickly escaping after dissolving the supercritical fluid-soluble substances. Therefore, the fast dissolving oral tablets according to the present invention produced by using the supercritical fluid extraction process has a high initial investment but can allow for the mass production. In addition, the supercritical fluid-soluble substances extracted when producing can be recycled and the time for producing can be reduced so that it is economical. In addition, the modification of the tablets is little after the extraction process when producing the fast dissolving oral tablets according to the present invention so that it is preferable and also the material that is not harmful to the environment can be used as the supercritical fluid so that it has an advantage such that it is an eco-friendly process. In addition, the fast dissolving oral tablets can be energy-saving tablets because an excessive heat is also not used.

For producing of the tablets by tableting the mixture, 0.1∼1000 parts by weight of the pharmaceutically acceptable additives and 0.01∼100 parts by weight of the supercritical fluid-soluble substances may be included based on 1 part by weight of the active ingredients. The above amounts may be properly adjusted according to a type of the active ingredients. The pharmaceutically acceptable additives include an excipient, a binder, a lubricant, and the like, but are not limited thereto.

The excipient may use D-mannitol, sorbitol, xylitol, isomalt, glucose, maltodextrin, trehalose, dextrose, sucrose, fructose, maltose, maltitol, methylcellulose, microcrystalline cellulose, lactose, low substituted hydroxycellulose, carboxymethylcellulose, or alkali metallic salt of carboxymethylcellulose, carrageenan, galactomannan, tragacantha, pectin, chitosan, or derivatives of chitin, gum arabic, xanthan gum, alginate or alginic alkali metal, polymethacrylate or salt thereof, methacrylate copolymer, polyvinylalcohol, polyvinylpyrrolidone, copolymer of polyvinylpyrrolidone and vinylacetate, polypropyleneoxide or polymer thereof, and the like. The binder may use polyvinylpyrrolidone, low substituted hydroxypropylcellulose, hydroxypropylcellulose, and the like, and the lubricant may use magnasium stearate, sodium stearyl fumarate, silica dioxide or talc, and the like, but any additive known in the pharmaceutics field can be used by properly selecting by the person in the art.

The supercritical fluid-soluble substances may use fatty acid, ester of fatty acid, volatile or non-volatile alcohol or derivatives thereof, organic acid, low boiling point solid or semi-solid hydrocarbon, aromatic hydrocarbon aldehyde, or the combinations thereof, but is not limited thereto. For example, menthol, camphor, thymol, benzoic acid, eucalyptol, salicylic acid, salicylic acid methyl, naphthalene or derivative thereof, cetyl alcohol, stearate, stearate alcohol, polyethyleneglycol, vanillin or combinations thereof can be used.

A cosolvent can be further used together with the supercritical fluid when contacting the tablets tableted in the step for forming the fine pores with the supercritical fluid. When extracting the supercritical fluid-soluble substances by using the cosolvent together with the supercritical fluid, the solubility of the supercritical phase of the supercritical fluid-soluble substances can be increased or the extraction can be performed at lower temperature so that it is preferable in terms of the chemical stability of the drug. The cosolvent may include for example ethanol, methanol, ether, chloroform, acetone, dimethylchloride, water, or the combination thereof.

For forming the fine pores, the supercritical fluid may include supercritical carbon dioxide, supercritical nitrogen monoxide, supercritical acetylene, supercritical trifluoromethane, supercritical propane, supercritical ethylene, supercritical chloro fluoro carbon or supercritical xenon, and the like. The reaction for extracting the supercritical fluid-soluble substances by contacting the tablets tableted with the supercritical fluid can be performed at 10∼90 °C and 30∼400 bar.

The active ingredients that can be applied for the fast dissolving oral tablets according to the present invention may include any drug that is needed for the fast dissolving oral tablets, and for example may include an antipyretic analgesics, an antiulcer agent, a prokinetic agent, an antimicrobial agent, an anti-inflammatory agent, an anthelmintic, an antiviral agent, an anxiolytics, an antidepressant, an antiepileptic, an antipsychotic agent, a morphine derivative, a topical anesthetic, an antifungal agent, an anticoagulant, an anticonvulsant, an erectile dysfunction treating agent, an antihistaminic agent, a decongestant, an antidiarrhea agent, a diuretic agent, the combination thereof, and the like.

For example, the antipyretic analgesics and anti-inflammatory agent include acetaminophen, ibuprophen, dexiprophen, aspirin, tramadol, indomethasin, diclofenac, ketoprophen, celecoxib or pharmaceutically acceptable salts thereof, and the like; the antiulcer agent and prokinetic agent include Cimetidine, Famotidine, Ranitidine, Roxatidine, Omeprazole, Lansoprazole, Pantoprazole, Mosapride, Metoclopramide, Domperidone or pharmaceutically acceptable salts thereof, and the like; the antimicrobial agent and anti-inflammatory agent include beta-lactam antibiotic agent, tetracycline, chloramphenicol, sulfonamides, aminoglycosides, Tobramycin, and the like; the erectile dysfunction treating agent includes Sildenafil, Vardenafil, Udenafil, and the like; the anxiolytics includes Bupropion, Fluoxetine or Venlafaxine, and the like; the anticonvulsant includes Carbamazepine, Lamotrigine or Phenytoin, and the like; and the antipsychotic agent includes Olanzapine, Aripiprazole, Risperidone, Haloperidol or Chlorpromazine, and the like. In addition, the antiviral agent includes Acyclovir, Famciclover or Valaciclovir, and the like; the antidiarrhea agent includes Loperamide, and the like; and the antihistaminic agent includes phenylamine, Loratadine, Cetirizine, Levocetirizine or Fexofenadine, and the like.

The fast dissolving oral tablets according to the present invention can be dissolved within about 40 second in the mouth so that the fast dissolving property is very excellent. In addition, the fast dissolving oral tablets according to the present invention also can be made with the hardness more than 7kp so that it can be sufficiently stand against the vibration during the conveying and packaging process, and there is no concern about damage while putting it within the mouth after removal from the PTP packing by hand.

In addition, another aspect of the present invention provides a method for producing the fast dissolving oral tablets according to the present invention, in which the method includes:

producing the tablets by tableting the mixture containing the active ingredients, the pharmaceutically acceptable additives, and the supercritical fluid-soluble substances; and

forming the fine pores in the tablets by extracting the supercritical fluid-soluble substances by contacting the tablets with the supercritical fluid.

As mentioned above, the fast dissolving oral tablets according to the method of the present invention have a sufficient hardness and also fast dissolving property by forming the porous pores in the tablets by using the supercritical fluid extraction process, and can be quickly produced in quantity due to the use of the supercritical extraction process. In addition, the supercritical fluid-soluble substances extracted when producing can be recycled so that it is economical and also they have an eco-friendly advantage because of using the material that is not harmful to the environment as the supercritical fluid.

### BRIEF DESCRIPTION OF THE DRAWINGS

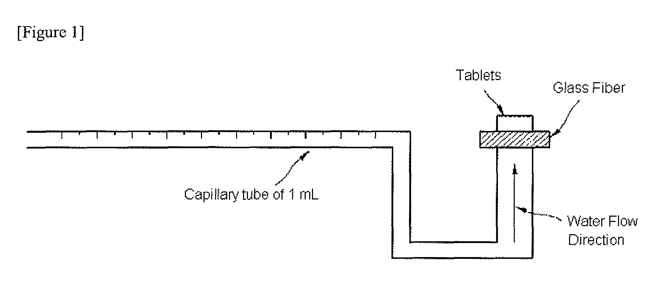

Fig. 1 is a figure showing a putting situation of a tablet to be measured on a measuring device of a water adsorption power by using a capillary tube filled with water, in which the measuring device is generally used for measuring the water absorption power of the tablet in Experiment Example 1.

### DESCRIPTION OF SPECIFIC EMBODIMENTS

Hereinafter, the present invention will be described in more detail according to the following Examples. However, the Examples are only for understanding the present invention, but the range of the present invention is not limited thereto in a sense.

**Comparative Example 1**

A general tablet having a total weight of tablet 100 mg and hardness of 7 kp was produced by using 90 mg D-mannitol as a sugar alcohol, 2.5 mg PVP k-30 as a binder, 5 mg cetyl alcohol as a supercritical fluid-soluble substances and 2.5 mg SiO₂ as a lubricant.

**Example 1**

A general tablet having a total weight of tablet 100 mg and hardness of 7 kp was produced by using 90 mg D-mannitol as a sugar alcohol, 2.5 mg PVP k-30 as a binder, 5 mg cetyl alcohol as a supercritical fluid-soluble substances and 2.5 mg SiO₂ as a lubricant. And then, the cetyl alcohol that is a supercritical fluid-soluble substance was purified and extracted by contacting the tablet with a supercritical fluid at 40 °C temperature and 80 bar pressure for 30 minutes to produce fast dissolving tablets having fine pores.

**Examples 2**

A general tablet having a total weight of tablet 100 mg and hardness of 7 kp was produced by using 90 mg methylcellulose as an excipient, 2.5 mg PVP k-30 as a binder, 5 mg cetyl alcohol as a supercritical fluid-soluble substances and 2.5 mg SiO₂ as a lubricant. And then, the cetyl alcohol that is a supercritical fluid-soluble substance was purified and extracted by contacting the tablet with a supercritical fluid at 40 °C temperature and 80 bar pressure for 30 minutes to produce fast dissolving tablets having fine pores. An extraction rate of the supercritical fluid-soluble substances was 50.4%.

**Example 3**

Fast dissolving tablets were produced by using the same method with Example 2, except that the general tablet was contacted to the supercritical fluid at 40 °C temperature and 80 bar pressure for 90 minutes in above Example 2. An extraction rate of the supercritical fluid-soluble substances was 101.0%.

**Example 4**

Fast dissolving tablets were produced by using the same method with Example 2, except that the general tablet was contacted to the supercritical fluid at 40 °C temperature and 120 bar pressure for 30 minutes in above Example 2. An extraction rate of the supercritical fluid-soluble substances was 97.8%.

**Example 5**

Fast dissolving tablets were produced by using the same method with Example 2, except that the general tablet was contacted to the supercritical fluid at 40 °C temperature and 160 bar pressure for 30 minutes in above Example 2. An extraction rate of the supercritical fluid-soluble substances was 100.0%, but it could be confirmed that a part of surface of the tablet in addition to the soluble substance was broken to loss.

**Comparative Example 2**

A general tablet was produced by homogenously mixing 325 mg acetaminophen as an active ingredient, 52 mg D-mannital as the sugar alcohol, 5 mg PVP k-30 as the binder, 1.5 mg SiO₂ as the lubricant, and 5 mg sodium stearyl fumarate per 1 tablet, and then tableting the mixed powder to be total weight 420 mg per 1 tablet and 9 kp hardness.

**Comparative Example 3**

A general tablet was produced by using the same method with the above Comparative Example 2, except that methylcellulose was used instead of D-mannitol as the sugar alcohol.

**Example 6**

A general tablet was produced by homogenously mixing 325 mg acetaminophen as an active ingredient, 52 mg D-mannitol as the sugar alcohol, 5 mg PVP k-30 as the binder, 1.5 mg SiO₂ as the lubricant, and 5 mg sodium stearyl fumarate per 1 tablet, and then tableting the mixed powder to be total weight 420 mg per 1 tablet and 9 kp hardness.

And then, the cetyl alcohol that is the supercritical fluid-soluble substance was purified and extracted by contacting the tablet with a supercritical fluid at 40 °C temperature and 80 bar pressure for 90 minutes to produce fast dissolving tablets having fine pores.

**Example 7**

A general tablet was produced by using the same method with the above Example 6, except that methylcellulose was used instead of D-mannitol as the sugar alcohol.

**Experiment Example 1: Test for Water Adsorption Power**

For the general tablet before extracting the supercritical fluid-soluble substances produced in the above Comparative Example 1 and the fast dissolving tablets after extracting the supercritical fluid-soluble substances produced in Examples 2 to 5, a water absorption power per a unit time was measured by using a measuring device of the water adsorption power by using a capillary tube filled with water, as shown in Fig 1, in which the measuring device is generally used for measuring the water absorption power of the tablet. A moving amount of water filled to the capillary tube per unit time was measured in mL/sec unit according to the water absorption power of tablet putted on a glass fiber.

The results were shown in the following Table 1.

[Table 1]

| | Com. Example 1 | Example 2 | Example 3 | Example 4 | Example 5 |
|---|---|---|---|---|---|
| Water Absorption Power (mL/sec | 0.002 | 0.0032 | 0.0054 | 0.0047 | 0.0050 |
| Time for Dissolving | 60 seconds | 55 seconds | 10 seconds | 30 seconds | 25 seconds |

As shown in the above Table 1, it could be known that the water absorption power in the case of the fast dissolving tablets according to the present invention was increased to about 2∼3 times as compared with the tablets before extracting the supercritical fluid-soluble substances by using the supercritical fluid.

**Experiment Example 2: Test for Dissolving**

For the general tablets before extracting the supercritical fluid-soluble substances produced in the above Comparative Examples 2 and 3, and the fast dissolving tablets after extracting the supercritical fluid-soluble substances produced in Examples 6 and 7, the time used for completely dissolving in the test tube filled with a certain amount of water was measured.

[Table 2]

| | Com. Example 2 | Example 6 | Com. Example 3 | Example 7 |
|---|---|---|---|---|
| Hardness (kp) | 9 | 9 | 9 | 9 |
| Content % | 99.7 | 99.12 | 99.8 | 99.06 |
| Extraction Rate % | - | 100.26 | - | 100.53 |
| Time for Dissolving (second) | 75 | 32 | 70 | 30 |

As shown in the above Table 2, it could be known that the fast dissolving tablets according to the present invention was characterized by having the dissolving time within about 30 seconds even though they include a high dose drug, and also the dissolving time of the supercritical fluid-soluble substance was significantly decreased as compared with the tablet before extracting the supercritical fluid-soluble substance by using the supercritical fluid.

While the present invention has been described with respect to the specific embodiments, it will be apparent to those skilled in the art that various changes and modifications may be made without departing from the spirit and scope of the invention as defined in the following claims.

## Claims

1. Fast dissolving oral tablets produced by using a method comprising:
producing the tablets by tableting the mixture containing active ingredients, pharmaceutically acceptable additives, and supercritical fluid-soluble substances; and
forming fine pores in the tablets by extracting the supercritical fluid-soluble substances by contacting the tablets with the supercritical fluid.

2. The Fast dissolving oral tablets of claim 1, wherein the supercritical fluid-soluble substances are fatty acid, ester of fatty acid, volatile or non-volatile alcohol or derivatives thereof, organic acid, low boiling point solid or semi-solid hydrocarbon, aromatic hydrocarbon aldehyde, or the combinations thereof.

3. The Fast dissolving oral tablets of claim 1, wherein the supercritical fluid-soluble substances are menthol, camphor, thymol, benzoic acid, eucalyptol, salicylic acid, salicylic acid methyl, naphthalene or derivative thereof, cetyl alcohol, stearate, stearate alcohol, polyethyleneglycol, vanillin or combinations thereof:

4. The Fast dissolving oral tablets of claim 1, wherein the step for forming the fine pores further uses a cosolvent together with the supercritical fluid.

5. The Fast dissolving oral tablets of claim 1, wherein the cosolvent is ethanol, methanol, ether, chloroform, acetone, dimethylchloride, water, or the combination thereof.

6. The Fast dissolving oral tablets of claim 1, wherein the step for forming the fine pores is performed at 10∼90 °C.

7. The Fast dissolving oral tablets of claim 1, wherein the step for forming the fine pores is performed at 30∼400 bar.

8. A method for producing fast dissolving oral tablets according to any one of claims 1 to 7, comprising:
producing the tablets by tableting the mixture containing active ingredients, pharmaceutically acceptable additives, and supercritical fluid-soluble substances; and
forming fine pores in the tablets by extracting the supercritical fluid-soluble substances by contacting the tablets with the supercritical fluid.
